# EUROPÄISCHE PATENTSCHRIFT

(11) **EP 0 686 843 B1**
(45) Veröffentlichungstag und Bekanntmachung des Hinweises auf die Patenterteilung: **15.01.1997**
(21) Anmeldenummer: 95107933.4
(22) Anmeldetag: 24.05.1995
(51) Int. Cl.: G01N 9/08, B65G 49/04

(54) **Vorrichtung zum automatischen Messen der Auftriebskraft keramischer Rohlinge in Quecksilber**
Device for automatic measurement of the buoyancy of green ceramic compacts in mercury
Dispositif pour la mesure automatique de la flottabilité d'ébauches céramiques en mercure

(30) Priorität: 08.06.1994 DE 4419960
(43) Veröffentlichungstag der Anmeldung: 13.12.1995
(73) Patentinhaber: IBERDITAN S.L., E-12006 Castellon (ES)
(72) Erfinder: Engmann, Dietrich, D-52134 Herzogenrath (DE)
(74) Vertreter: Biermann, Wilhelm, Dr.-Ing.

(56) Entgegenhaltungen:
- EP-A- 0 371 172
- EP-A- 0 392 593
- ES-A- 2 005 099
- ES-A- 2 009 165
- GB-A- 1 094 207
- US-A- 3 991 619
- PATENT ABSTRACTS OF JAPAN vol. 007 no. 270 (P-240) ,2.Dezember 1983 & JP-A-58 148940 (NIHON DENSHI KIKI KK) 5.September 1983,

## Beschreibung

Die Erfindung betrifft eine Vorrichtung zum Messen der Auftriebskraft einer plattenförmigen keramischen Probe in Quecksilber, mit einem das Quecksilber enthaltenden Behälter, einem unterhalb des Quecksilberspiegels liegenden, mit einem Kraftmesser verbundenen Kraftaufnehmer, sowie einer die Probe in das Quecksilber eintauchenden, unter dem Kraftaufnehmer positionierenden und wieder aus dem Quecksilberbad entfernenden Transporteinrichtung.

Bei der Herstellung von keramischen Körpern, insbesondere Fliesen, ist die Kenntnis der Verteilung der Dichte über der gesamten Fläche der keramischen Körper von großer Bedeutung. In verschiedenen Phasen des Herstellungsprozesses, nämlich beim Trocknen der gepreßten Rohlinge, beim Glasieren und beim Brennen der Fliesen, ist die Dichte ein entscheidendes Kriterium für die Qualität des entstehenden Produktes. Besondere Bedeutung hat die Dichte beim Brennvorgang, bei dem die Fliesen in den modernen Brennöfen in kurzer Zeit auf sehr hohe Temperaturen erhitzt werden. Bei ungleichmäßiger Verteilung der Dichte entstehen hauptsächlich zwei Probleme, die einen nachteiligen Einfluß auf die Qualität haben: An Stellen zu hoher Dichte blähen die Körper auf und/oder es bildet sich an diesen Stellen ein sogenannter schwarzer Kern, und an Stellen mit zu geringer Dichte entsteht eine größere Schwindung. Sowohl eine stellenweise zu hohe Dichte als auch eine zu geringe Dichte bei den gepreßten Rohlingen führen damit beim Brennen zu Oberflächendeformationen infolge unterschiedlich starker Schwindung.

In modernen Fertigungsprzessen wird deshalb immer mehr dazu übergegangen, bei den Rohlingen, das heißt nach dem Pressen, die Verteilung der Dichte systematisch zu kontrollieren. Zu diesem Zweck wird während der Produktion in bestimmten Abständen ein Fliesenrohling mittels einer geeigneten Schneidvorrichtung in eine Anzahl von Proben zerteilt, von denen die sogenannte "scheinbare Dichte" bestimmt wird. Die einzelnen Proben der Fliese entsprechen bestimmten Bereichen der Pressform, in der der Fliesenrohling gepreßt wurde. Die Schüttmenge des Rohmaterials wird in den einzelnen Bereichen der Pressform in Abhängigkeit von der scheinbaren Dichte der jeweils zugeordneten Proben durch Ändern der Pressenparameter von Hand justiert oder automatisch geregelt. Das Verfahren zur automatischen Regelung der Dichte von keramischen Preßkörpern ist aus der EP 0392593 A2 bekannt.

Die Ermittlung der "scheinbaren Dichte" der Probenkörper erfolgt in der Weise, daß zum einen das Gewicht der Probenkörper und zum anderen durch Eintauchen der Probenkörper in Quecksilber der Auftrieb in Quecksilber ermittelt wird. Aus diesen beiden Meßwerten läßt sich die "scheinbare Dichte" errechnen.

Eine Vorrichtung der eingangs genannten Art für die Ermittlung der Auftriebskraft in Quecksilber ist aus der italienischen Patentschrift 1.219.039 sowie aus dem entsprechenden spanischen Dokument ES-A-2 009 165 bekannt. Bei dieser bekannten Vorrichtung ist unmittelbar oberhalb des Quecksilberbehälters eine entfernbare Trägerplatte angeordnet, auf die die Probe aufgelegt oder aufgeschoben wird. Durch Aussparungen in der Trägerplatte greifen zwei an einer Haltevorrichtung befestigte Tragbügel, die unter die Probe greifen und die Probe von der Trägerplatte abheben. Die Trägerplatte wird dann weggeschwenkt, und die Haltevorrichtung mit den beiden die Probe festklemmenden Tragbügeln wird in das Quecksilberbad abgesenkt. Die Haltevorrichtung, die während des übernahmevorgangs der Probe von der Trägerplatte und während des Absenkvorgangs der Probe in das Quecksilberbad insgesamt von dem Kraftaufnehmer getragen und betätigt wird, wird nach dem Eintauchen der Probe in das Quecksilberbad auf eine von der Fliesendicke unabhängige Höhe gebracht. Dadurch wird der Auftrieb der Haltevorrichtung zu einer konstanten Größe, so daß der sich anschließende Meßvorgang den korrekten Meßwert des Auftriebs des Probenkörpers angibt.

Bei dieser bekannten Vorrichtung sind also verschiedene nacheinander ablaufende Schritte für die Positionierung der Probe in dem Quecksilberbad erforderlich, was einen gewissen Zeitbedarf erfordert. Durch das senkrechte Eintauchen der Probe in das Quecksilber werden außerdem Bewegungen des Quecksilbers hervorgerufen, die erst vollständig ausklingen müssen, bevor der Meßvorgang beginnen kann, da der Stillstand des Systems Voraussetzung für ein exaktes Wägen ist. Der Meßvorgang für eine Probe benötigt daher bei dieser Vorrichtung insgesamt eine verhältnismäßig lange Zeit. Für die Dichteermittlung einer ganzen Fliese müssen aber eine Reihe von Proben, beispielsweise sechzehn Stück, nacheinander auf diese Weise gemessen werden, bevor Aussagen über die Dichteverteilung gemacht und korrigierende Maßnahmen beim Füllen der Preßformen eingeleitet werden können. Die Zeitabstände für eine Korrektur der Presse oder für eine Prozeßregelung sind daher bei Einsatz der bekannten Vorrichtung verhältnismäßig lang.

Der Erfindung liegt die Aufgabe zugrunde, eine Vorrichtung der eingangs genannten Art zu entwickeln, die es gestattet, den Wägevorgang zur Bestimmung des Auftriebs im Quecksilberbad in wesentlich kürzerer Zeit durchzuführen. Die Vorrichtung soll sich insbesondere für die Integrierung in eine automatische Linie für die Bestimmung der scheinbaren Dichte an aufeinanderfolgenden Proben eignen, so daß der Vorgang insgesamt wesentlich abgekürzt wird und dadurch die Reaktionszeiten für die Justierung der Presse und/oder für die Prozessregelung verbessert werden.

Gemäß der Erfindung wird diese Aufgabe dadurch gelöst, daß der Kraftaufnehmer auch während des Positionierungsvorgangs der Proben im Quecksilberbad verbleibt, und daß die Transportvorrichtung einen von dem Kraftaufnehmer unabhängigen, die plattenförmigen Proben seitlich neben dem Kraftaufnehmer unter einem spitzen Winkel der Plattenebene zur Badoberfläche in das Quecksilberbad eintauchenden und an den Kraftaufnehmer übergebenden Zuförderer und einen die plattenförmigen Proben von dem Kraftaufnehmer übernehmenden und seitlich neben dem Kraftaufnehmer unter einem spitzen Winkel der Plattenebene zur Badoberfläche aus dem Quecksilberbad entfernenden Abförderer umfaßt.

Bei der erfindungsgemäßen Vorrichtung werden also Bewegungen des Quecksilberbades durch Eintauchen des Kraftaufnehmers in das Quecksilber vermieden, so daß die Zeiten bis zur jeweiligen Stillstandsituation der Meßvorrichtung wesentlich verkürzt werden. In demselben Sinne wirkt das Fördersystem, durch das die Proben seitlich neben dem Kraftaufnehmer unter einem zur Badoberfläche spitzen Winkel in das Quecksilber eingetaucht werden, wodurch ebenfalls die durch das Eintauchen der Proben bedingten Schwingungen des Quecksilberbades auf ein Minimum reduziert werden. Infolge dieser Anordnung läßt sich die erfindungsgemäße Vorrichtung in besonders einfacher Weise automatisieren und in eine automatische Linie integrieren,so daß sich hierdurch eine erhebliche Verkürzung des gesamten Meßvorganges und damit eine wesentliche Verbesserung des Regelungsprozesses insgesamt und infolgedessen eine deutliche Steigerung des Ausbringens einer automatischen Fertigungslinie und Verbesserung der Qualität des Endprodukts erreichen läßt.

Das Fördersystem zum Eintauchen der Proben in das Quecksilberbad, zum Positionieren und zum überführen der Proben aus dem Quecksilberbad auf einen nachfolgenden Förderer kann grundsätzlich verschieden ausgebildet sein. So kann es zum Beispiel aus einem Greifersystem bestehen, bei dem die Proben von zangenartigen Greifern erfaßt werden, die durch ein geeignetes mechanisches System in der gewünschten Bahn geführt werden.

Gemäß einer besonders zweckmäßigen Ausführungsform besteht der Zuförderer aus einem unter einem spitzen Winkel in das Quecksilberbad eintauchenden Förderband, und der Abförderer aus einem unter einem spitzen Winkel aus dem Quecksilberbad auftauchenden Förderband. Vorteilhafterweise sind die Förderbänder des Zuförderers und des Abförderers jeweils unter einem Winkel von 20 bis 50 Grad zur Badoberfläche angeordnet, und vorzugsweise unter einem Winkel von 30 bis 45 Grad.

In zweckmäßiger Weiterbildung ist dem Förderband des Zuförderers und dem Förderband des Abförderers oberhalb von diesen jeweils ein weiteres Förderband zugeordnet, wobei die einander zugeordneten Förderbänder synchron zueinander laufen und die Proben zwischen sich einschließen.

Der Horizontaltransport im Bereich des Kraftaufnehmers innerhalb des Quecksilberbades erfolgt in besonders vorteilhafter Ausgestaltung der Erfindung ebenfalls durch ein Förderband, und zwar derart, daß die Probenkörper durch die Auftriebskraft von unten gegen das Förderband gedrückt werden. Dieses Förderband kann beispielsweise ein integraler Bestandteil des Kraftaufnehmers sein. Es ist jedoch auch möglich, dieses Förderband unabhängig von dem Kraftaufnehmer zu lagern und zu führen, und es mitsamt der von unten hiergegen anliegenden Probe nur im Zeitpunkt der eigentlichen Auftriebsmessung gegen den Kraftaufnehmer zur Anlage zu bringen.

Die Ausbildung des Fördersystems in Form von Förderbändern hat ferner den besonderen Vorteil, daß problemlos Proben unterschiedlicher Abmessungen durch das Quecksilberbad transportiert und ausgewertet werden können. Dadurch wird es möglich, die Fliesen insgesamt, das heißt lückenlos, in Probenkörper zu unterteilen und auszuwerten, ohne daß wie bei der bekannten Vorrichtung zwischen den Probekörpern streifenförmige Bereiche verbleiben, die nicht ausgewertet werden konnten. Außerdem erlaubt diese Ausbildung des Fördersystems die Auswertung kleinerer Proben, und damit eine größere Auflösung der Oberfläche der Fliesen, das heißt eine größere Anzahl von Proben und auf diese Weise eine genauere Messung der Dichteverteilung.

Durch die erfindungsgemäße Vorrichtung wird es außerdem möglich, den Kraftaufnehmer oder sogar die Meßvorrichtung insgesamt unterhalb des Quecksilberspiegels anzuordnen. Auf diese Weise kann die Meßgenauigkeit der Vorrichtung weiter erhöht werden, weil die Eintauchtiefe dieser Teile sich beim Meßvorgang sich nicht verändert und eine darauf beruhende Veränderung der Auftriebskräfte dadurch sicher vermieden wird.

Weitere Merkmale, Einzelheiten und Vorteile der Erfindung ergeben sich aus der nachfolgenden Beschreibung bevorzugter Ausführungsbeispiele der Erfindung anhand der Zeichnungen.

Von den Zeichnungen zeigt, jeweils in schematischer Darstellung,

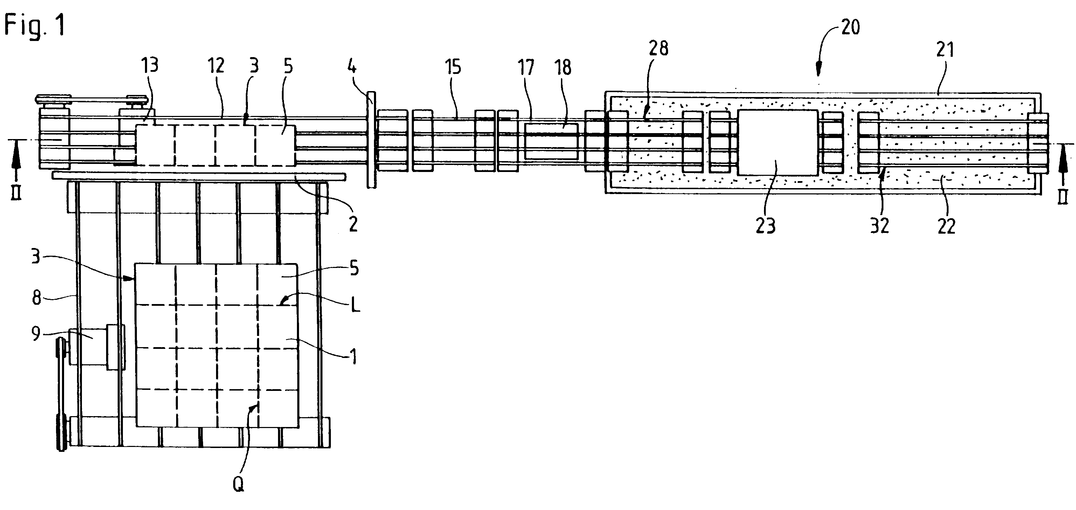

- Fig. 1: eine automatische Linie zur Bestimmung der Dichteverteilung innerhalb eines Fliesenrohlings, in der Aufsicht;

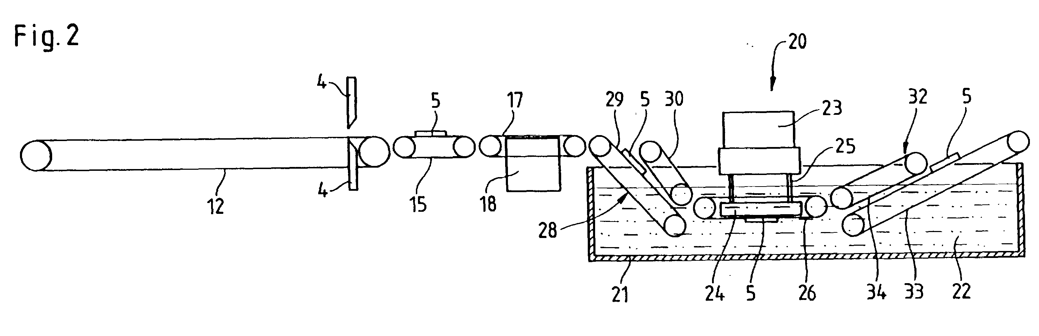

- Fig. 2: einen Schnitt entlang der Linie II-II der Fig.1;
- Fig. 3: eine andere Ausführungsform der eigentlichen Auftriebsmeßvorrichtung in Form eines senkrechten Längsschnitts, und
- Fig. 4: einen Schnitt entlang der Linie IV-IV in Fig.3, jedoch bei angehobener Stellung des Förderbandes.

Die automatische Linie zur Bestimmung der Dichteverteilung in einem Fliesenrohling 1 umfaßt eine erste Schneideinrichtung 2, durch die der Fliesenrohling 1 entlang den Längsschnittlinien L in beispielsweise vier gleichbreite Streifen 3 unterteilt wird, und eine zweite Schneideinrichtung 4, durch die jeder Streifen 3 entlang den Querschnittlinien Q in vier Proben 5 unterteilt wird.

Die Schneideinrichtung 2 befindet sich am Ende eines Horizontalförderers 8, auf den die Fliesenrohlinge 1 aufgelegt werden und der die Rohlinge zur Schneideinrichtung 2 fördert. Der Horizontalförderer 8 wird durch einen Antriebsmotor 9 angetrieben, beispielsweise einen Schrittmotor, der eine genaue Positionierung des Fliesenrohlings 1 gestattet. Nach Ausführung des Schnitts durch die Schneideinrichtung 2 werden die abgeschnittenen Streifen 3 durch den Horizontalförderer 8 auf den Förderer 12 übergeben, an dessen Ende sich die Querschneideeinrichtung 4 befindet. Auch der Förderer 12 wird durch einen exakt steuerbaren Antrieb 13, insbesondere einen Schrittmotor, angetrieben, der eine genaue Positionierung des Streifens 3 erlaubt. Auf diese Weise wird der Streifen 3 in beispielsweise vier gleichgroße Proben 5 unterteilt, die nun der Reihe nach auf den Förderer 15 übergeben werden.

Von dem Förderer 15 werden die Proben 5 auf einen Förderer 17 übergeben, der Teil einer Wägestation ist, in der durch eine Waage 18 das Gewicht der Proben 5 ermittelt wird. Das Messen des Gewichts kann dabei während des kontinuierlichen Transports der Proben oder während einer kurzzeitigen Unterbrechung des Transports am Meßsystem erfolgen. Als Meßsysteme zum Wägen der Proben können übliche elektronische Waagen oder bekannte Druck- oder Kraftsensoren mit entsprechender elektronischer Auswertung eingesetzt werden.

Nach Verlassen der Wägestation 18 werden die Proben 5 von dem Förderer 17 an die Auftriebsmeßvorrichtung 20 übergeben. Die Auftriebsmeßvorrictung 20 umfaßt einen Behälter 21, in dem sich das Quecksilberbad 22 befindet. Außerhalb des Quecksilberbades 22 befindet sich die eigentliche Meßvorrichtung 23. Der Kraftaufnehmer 24 ist innerhalb des Quecksilberbades 22 angeordnet und ist über Stangen 25 mit der Meßvorrichtung 23 verbunden. In dem hier dargestellten Fall ist an dem Kraftaufnehmer 24 ein endloses Förderband 26 einschließlich des Antriebs für dieses Förderband angeordnet.

Bei der Auftriebsmessung ist die Höhe des Quecksilberspiegels von dem Volumen der eingetauchten Probenkörper abhängig. Infolgedessen können die Meßergebnisse dadurch beeinflußt werden, daß die auf die eingetauchten Teile der Stangen 25 einwirkenden Auftriebskräfte sich geringfügig ändern. Um diesen Einfluß zu minimieren, weisen die Stangen 25 einen sehr kleinen Durchmesser auf, so daß die Volumenänderung der eingetauchten Stangenabschnitte praktisch vernachlässigbar wird.

Ein stromaufwärts des eingetauchten Kraftaufnehmers 24 angeordneter Förderer 28 übernimmt die Proben 5 von dem Förderer 17. Der Förderer 28 bildet den Zuförderer zum Kraftaufnehmer 24. Er besteht aus einem unteren endlosen Förderband 29 und einem in geringem Abstand oberhalb von diesem und im wesentlichen parallel dazu angeordneten weiteren endlosen Förderband 30. Die Förderbänder 29 und 30 sind synchron angetrieben und greifen die Proben 5 zwischen sich. Die Förderbänder 29 und 30 sind unter einem spitzen Winkel von etwa 30 bis 50 Grad zur Badoberfläche angeordnet, und der untere Teil der Förderbänder taucht in das Quecksilber ein. Das vordere Ende des Förderbandes 29 befindet sich in der Nähe der hinteren Umkehrrolle des Förderbandes 26. Sobald die in das Quecksilberbad eingetauchte Probe 5 von dem oberen Förderband 30 freigegeben wird, legt sich die Probe 5 unter der Wirkung der Auftriebskraft von unten gegen das Untertrum des Förderbandes 26 an und wird nun von diesem weitertransportiert. Wenn die Probe 5 die gewünschte Stelle unter dem Kraftaufnehmer 24 durchläuft, wird die Messung entweder während eines kurzzeitigen Anhaltens des Förderbandes oder während des Durchlaufens unter Bildung eines Mittelwerts durchgeführt.

Nach Durchführung der Auftriebsmessung wird der Antrieb für das Förderband 26 wieder eingeschaltet, und die Probe 5 wird an den Abförderer 32 übergeben. Der Abförderer 32 umfaßt wiederum ein unteres endloses Förderband 33 und ein in geringem Abstand oberhalb von diesem im wesentlichen parallel hierzu angeordneten endlosen Förderband 34, wodurch im Bereich des Quecksilberbades die Proben 5 zwischen den beiden Förderbändern gegriffen werden. Am Ende des Förderbandes 33 fallen die Proben 5, die nun nicht mehr benötigt werden, entweder in einen bereitstehenden nicht dargestellten Behälter, oder sie werden einem weiteren, ebenfalls nicht dargestellten Förderer übergeben.

Die Meßergebnisse der Waage 18 und der Auftriebsmeßvorrichtung 23 werden in einer nicht dargestellten elektronischen Schaltung ausgewertet und hieraus die Dichtewerte errechnet. Da die Proben 5 durch die automatischen Schneideinrichtungen immer in der gleichen vorgegebenen Reihenfolge die Meßvorrichtungen durchlaufen, ist die Auswertung und die Zuordnung der einzelnen Meßwerte zu den entsprechenden Teilbereichen der Presse automatisch sichergestellt. Der gesamte Meßzyklus für sämtliche Proben eines Fliesenrohlings nimmt nur noch einen Bruchteil der Zeit der bekannten Vorrichtungen ein, so daß eine sehr viel wirksamere Prozessregelung möglich ist.

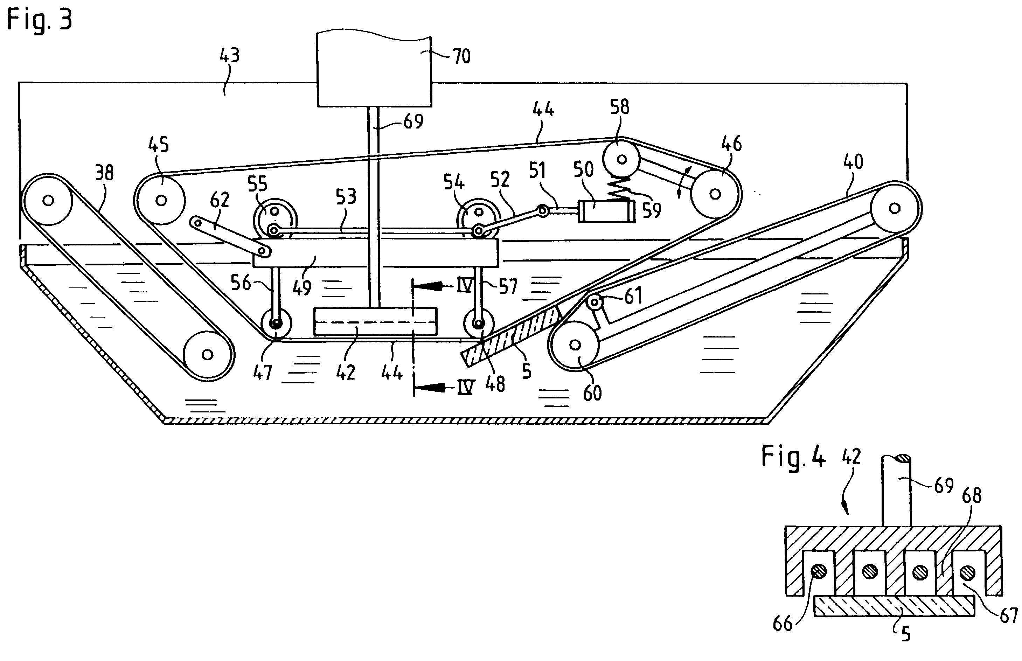

Eine andere Ausführungsform der Auftriebsmeßvorrichtung ist in Fig.3 und 4 dargestellt. Bei dieser Ausführungsform sind ebenfalls schräg in das Quecksilberbad eintauchende Zuförderer und Abförderer vorgesehen, die ein unteres endloses Förderband 38 auf der Zuförderseite und ein unteres endloses Förderband 40 auf der Abförderseite aufweisen. Die hiermit zusammenwirkenden oberen Förderbänder sind jedoch in diesem Fall mit dem Förderband, das die Positionierung der Probe unter dem Kraftaufnehmer 42 vornimmt, zu einem einzigen Förderband 44 zusammengefaßt. Dieses kombinierte Förderband 44 ist unabhängig von dem Kraftaufnehmer 42 gelagert, und zwar auf den beiden an dem Maschinenrahmen 43 angeordneten Umlenkrollen 45 und 46, sowie den beiden in Höhe des Kraftaufnehmers 42 in senkrechter Richtung beweglich gelagerten Umlenkrollen 47 und 48. Um die Achse der Umlenkrolle 46 schwenkbar ist eine Spannrolle 58 gelagert, die unter der Wirkung einer Feder 59 nach oben gedrückt wird. Dadurch wird das Förderband 44 unter Spannung gehalten.

In der Transportsituation befinden sich die beiden unteren Umlenkrollen 47, 48 in der dargestellten abgesenkten Position. In dieser abgesenkten Position hat die von dem Förderband 44 gehaltene und mit dem Förderband 44 durchlaufende Probe keinen Kontakt mit dem Kraftaufnehmer 42, jedoch verlaufen in dieser Position die Bandabschnitte zwischen den Umlenkrollen 45 und 47 einerseits und zwischen den Umlenkrollen 46 und 48 andererseits derart benachbart zu den zugehörigen Förderbändern 38 bzw. 40, daß sie gemeinsam mit diesen den Transport der Proben zum Kraftaufnehmer 42 hin bzw. von diesem weg vornehmen.

Die unteren Umlenkrollen 47 und 48 sind über Stangen 56 bzw. 57 fest an dem Rahmen 49 angeordnet. Der Rahmen 49 ist in dem Quecksilberbad schwimmend gelagert und lediglich durch eine Lasche 62 geführt, die ihrerseits an ihrem anderen Ende an dem Maschinenrahmen 43 schwenkbar gelagert ist. Der Rahmen 49 wird durch die Summe der Auftriebskräfte und der durch das Förderband 44 senkrecht nach oben gerichteten Kraftkomponente gegen die Räder 54 und 55 gedrückt, die ihrerseits an dem Maschinenrahmen 43 exzentrisch gelagert sind. Die Räder 54 und 55 sind jeweils mit einem Spurkranz 65 versehen, die den Rahmen 49 seitlich übergreifen und auf diese Weise für die seitliche Führung des schwimmend gelagerten Rahmens 49 sorgen.

Wenn der Zylinder 50, der ebenfalls an dem Maschinenrahmen 43 angeordnet ist, die exzentrisch gelagerten Räder 54 über die Kolbenstange 51 und die Stangen 52 und 53 verschwenkt, rollen die Räder 54 und 55 nach oben schwenkend auf dem Rahmen 49 ab. Infolge der Auftriebskräfte und der nach oben gerichteten Zugkräfte des Förderbandes 44 bewegt sich der Rahmen 43 mit den Umlenkrollen 47 und 48 nach oben. Dabei dringen, wie aus Fig. 4 hervorgeht, die einzelnen das Förderband 44 bildenden Riemen 66 in entsprechende Aussparungen 67 in dem Kraftaufnehmer 42 ein und übergeben dabei den Probenkörper an den Kraftaufnehmer 42. Die Riemen 66 des Förderbandes 44 laufen dabei zwischen den Rippen 68 des Kraftaufnehmers 42 weiter, so daß ein Anhalten des Riementriebes nicht erforderlich ist.

Dem unteren Abförderband 40 wird durch eine in der Nähe der unteren Umlenkrolle 60 angeordnete Rolle 61 auf seinem dem Kraftaufnehmer 42 benachbarten Teilabschnitt eine stärkere Neigung erteilt, wodurch das Einlaufen der Proben 5 zwischen die Förderbänder und das Ergreifen der Proben 5 erleichtert wird. Beim Einlaufen der Probenkörper gibt das obere Förderband 44 gegen die Wirkung der Feder 59 nach und sorgt dadurch für den nötigen Andruck gegen das untere Förderband 40.

Die Messung der Auftriebskraft erfolgt, wie bereits erwähnt, wenn die Probe 5 unter dem Kraftaufnehmer 42 positioniert ist bei angehobener Stellung des Rahmens 49. Die auf den Kraftaufnehmer 42 einwirkenden Kräfte werden über die Stange 69 auf die Meßvorrichtung 70 übertragen, die den jeweiligen Meßwert in Form geeigneter elektrischer Signale an die elektronische Auswerteeinheit weitergibt.

## Patentansprüche

1. Vorrichtung zum Messen der Auftriebskraft einer plattenförmigen keramischen Probe in Quecksilber, mit einem das Quecksilber enthaltenden Behälter, einem unterhalb des Quecksilberspiegels liegenden, mit einem Kraftmesser verbundenen Kraftaufnehmer, sowie einer die Probe in das Quecksilber eintauchenden, unter dem Kraftaufnehmer positionierenden und wieder aus dem Quecksilberbad entfernenden Transporteinrichtung, **dadurch gekennzeichnet**, daß der Kraftaufnehmer (24;42) auch während des Positioniervorgangs der Proben (5) im Quecksilberbad (22) verbleibt, und daß die Transporteinrichtung einenvon dem Kraftaufnehmer (24;42) unabhängigen, die plattenförmigen Proben (5) seitlich neben dem Kraftaufnehmer (24;42) unter einem spitzen Winkel der Plattenebene zur Badoberfläche in das Quecksilberbad (22) eintauchenden und an den Kraftaufnehmer (24;42) übergebenden Zuförderer und einen die plattenförmigen Proben (5) von dem Kraftaufnehmer (24;42) übernehmenden und seitlich neben dem Kraftaufnehmer (24;42) unter einem spitzen Winkel der Plattenebene zur Badoberfläche aus dem Quecksilberbad (22) entfernenden Abförderer umfaßt.

2. Vorrichtung nach anspruch 1, dadurch gekennzeichnet, daß der Zuförderer und der Abförderer aus einem Greifersystem mit zangenartigen Greifern besteht.

3. Vorrichtung nach Anspruch 1, dadurch gekennzeichnet, daß der Zuförderer aus einem unter einem spitzen Winkel in das Quecksilberbad (22) eintauchenden Förderband (29;38), und der Abförderer aus einem unter einem spitzen Winkel aus dem Quecksilberbad (22) auftauchenden Förderband (33;40) bestehen.

4. Vorrichtung nach Anspruch 3, dadurch gekennzeichnet, daß das Förderband (29;38) des Zuförderers und das Förderband (33;40) des Abförderers unter einem Winkel von 20 bis 50 Grad, und vorzugsweise unter einem Winkel von 30 bis 45 Grad zur Badoberfläche des Quecksilberbades (22) angeordnet sind.

5. Vorrichtung nach Anspruch 3 oder 4, dadurch gekennzeichnet, daß dem Förderband (29;38) des Zuförderers und dem Förderband (33;40) des Abförderers oberhalb von diesen jeweils ein weiteres Förderband (30,32;40) zugeordnet ist, wobei die einander zugeordneten Förderbänder synchron zueinander laufen und die Proben zwischen sich einschließen.

6. Vorrichtung nach einem der Ansprüche 3 bis 5, dadurch gekennzeichnet, daß an dem Kraftaufnehmer (24) ein horizontales, die Proben (5) von dem Zuförderer (28) übernehmendes und nach dem Meßvorgang an den Abförderer (32) übergebendes Förderband (26) angeordnet ist.

7. Vorrichtung nach Anspruche 6, dadurch gekennzeichnet, daß das Förderband (26) des Kraftaufnehmers (24) einschließlich der Lagerung und des Antriebsmechanismus insgesamt vollständig in das Quecksilberbad (22) eingetaucht sind.

8. Vorrichtung nach Anspruch 7, dadurch gekennzeichnet, daß die in das Quecksilberbad (22) eingetauchte, das Förderband (26) sowie die Lagerung und den Antrieb des Förderbandes (26) umfassende Einheit mit der oberhalb des Quecksilberbades (22) angeordneten Meßvorrichtung (23) über verhältnismäßig dünne, und deshalb bei Veränderung der Eintauchtiefe nur sehr geringfügige Veränderungen der Auftriebskräfte bewirkende Stangen (25) verbunden ist.

9. Vorrichtung nach Anspruch 7, dadurch gekennzeichnet, daß auch die Meßvorrichtung und die den Kraftaufnehmer mit der Meßvorrichtung verbindenden Elemente insgesamt vollständig in das Quecksilber eingetaucht sind.

10. Vorrichtung nach einem der Ansprüche 3 bis 5, dadurch gekennzeichnet, daß das den Zuförderer bildende Förderband (38) und das den Abförderer bildende Förderband (40) ein zusammenhängendes, unter dem Kraftaufnehmer vorbeilaufendes Fördersystem aus einzelnen Transportriemen (66) bilden, und daß der Kraftaufnehmer (42) auf seiner Unterseite mit Aussparungen (67) versehen ist, in die nach einer vertikalen Relativbewegung zwischen dem Kraftaufnehmer (42) und den Transportriemen (66) im Anschluß an die Positionierung der Probe (5) beim Meßvorgang die Transportriemen (66) eindringen, wobei sich die Probe (5) gegen die die Aussparungen (67) begrenzenden Rippen (68) des Kraftaufnehmers (42) anlegt.

11. Vorrichtung nach einem oder mehreren der Ansprüche 1 bis 10, dadurch gekennzeichnet, daß sie in eine automatische Linie integriert ist, die eine vor- oder nachgeschaltete Wägestation (17,18) zur Ermittlung des Gewichts des Probenkörpers (5) umfaßt.

12. Vorrichtung nach Anspruch 11, dadurch gekennzeichnet, daß sie in eine automatische Linie integriert ist, die am Anfang der Linie eine automatische Schneideinrichtung (2,4) zum Unterteilen keramischer Fliesen oder gepreßter keramischer Fliesenrohlinge in einzelne Probenkörper (5) umfaßt.

## Claims

1. A device for measuring the upthrust force of a plate-like ceramic sample in mercury, comprising a container for the mercury, a force pick-up placed underneath the surface of the mercury and connected with a force measuring means and a shifting means for plunging the sample into the mercury, positioning same underneath the force pick-up and removing it again from the mercury bath, characterized in that the force pick-up (24; 42) also remains in the mercury bath (22) during the operation of positioning the samples (5) and in that the shifting means comprises a supply conveyor which is independent from the force pick-up (24; 42), plunges the plate-like samples (5) laterally adjacent to the force pick-up (24; 42) at an acute angle of the plane of the plate to the bath surface into the mercury bath (22) and transfers the force pick-up (24; 42), and a removal conveyor for receiving the plate-like samples (5) from the force pick-up (24; 42) and removing same laterally adjacent to the force pick-up (24; 42) at an acute angle of the plane of the plate to the bath surface out the mercury bath (22).

2. The device as claimed in claim 1, characterized in that the supply conveyor and the removal conveyor comprise a gripping system with tongs-like grippers.

3. The device as claimed in claim 1, characterized in that the supply conveyor comprises a conveyor belt (29; 38) dipping at an acute angle in the mercury bath (22) and the removing means comprises a conveyor belt (33; 40) emerging at an acute angle out of the mercury bath (22).

4. The device as claimed in claim 3, characterized in that the conveyor belt (29; 38) of the supply conveyor and the conveyor belt (33; 40) of the removing means are arranged at an angle of 20 to 50 degrees and preferably at an angle of 30 to 45 degrees to the surface of the mercury bath (22).

5. The device as claimed in claim 3 or in claim 4, characterized in that a respective further conveyor belt (30, 32; 40) is associated with conveyor belt (29; 38) of the supply conveyor and with the conveyor belt (33; 40) of the removal conveyor above same, the mutually associated conveyor belts running synchronously with one another and encompassing the samples between them.

6. The device as claimed in any one of the claims 3 through 5, characterized in that at the force pick-up (24) a horizontal conveyor belt (26) is arranged receiving the samples (5) from the supply conveyor (28) and transferring same to the removal conveyor (32) after the measuring operation.

7. The device as claimed in claim 6, characterized in that together with the bearing means and the drive mechanism the conveyor belt (26) of the force pick-up (24) is as a whole completely submersed in the mercury bath (22).

8. The device as claimed in claim 7, characterized in that the unit submersed in the mercury bath (22) and comprising the conveyor belt (26) together with the bearing means and the drive of the conveyor belt (26) is connected with the measuring device (23), arranged above the mercury bath (22), by means of rods (25) which are relatively thin and consequently only cause extremely minor changes in the upthrust forces.

9. The device as claimed in claim 7, characterized in that elements connecting the measuring device and the force pick-up with the measuring device are as a whole completely submersed in the mercury.

10. The device as claimed in any one of the claims 3 through 5, characterized in that the conveyor belt (38) forming the supply conveyor and the conveyor belt (40) forming removal conveyor form a unitary conveying system running past underneath the force pick-up and constituted by individual conveyor belts (66) and in that on the lower side thereof the force pick-up (42) is provided recesses (67) into which, after a relative vertical movement between the force pick-up (42) and the conveyor belts (66), following positioning of the sample (5) in the measuring operation the conveyor belts (66) fit, the sample (5) then assuming a position against the ribs (68) delimiting the recesses (67) of the force pick-up (42).

11. The device as claimed in any one or more of the claims 1 through 10, characterized in that same is integrated in an automatic production line, which comprises a weighing station (17, 18) for the determination of the weight of the sample body (5).

12. The device as claimed in claim 11, characterized in that same is integrated in an automatic production line, which at the start of the line comprises an automatic cutting device (2, 4) for the division up of ceramic tiles or pressed ceramic tile blanks to form separate sample bodies (5).

## Revendications

1. Dispositif pour mesurer la flottabilité d'une éprouvette de céramique en forme de plaquette dans du mercure, comportant un récipient contenant le mercure, un transducteur de force situé en dessous du niveau du mercure et relié à un dynamomètre, ainsi qu'un dispositif transporteur immergeant l'éprouvette dans le mercure, la positionnant sous le transducteur de force et la ressortant du bain de mercure, caractérisé en ce que le transducteur de force (24; 42) reste dans le bain de mercure (22) également pendant l'opération de positionnement des éprouvettes (5), et que le dispositif transporteur comprend un transporteur d'amenée indépendant du transducteur de force (24; 42), qui immerge les éprouvettes en forme de plaquette (5) dans le bain de mercure (22) latéralement à côté du transducteur de force (24; 42) sous un angle aigu que forme le plan de la plaquette avec la surface du bain et les délivre au transducteur de force (24; 42) et un transporteur d'évacuation qui reprend les éprouvettes en forme de plaquette (5) du transducteur de force (24; 42) et les retire du bain de mercure (22) latéralement à côté du transducteur de force (24; 42) sous un angle aigu que forme le plan de la plaquette avec la surface du bain.

2. Dispositif suivant la revendication 1, caractérisé en ce que le transporteur d'amenée et le transporteur d'évacuation sont constitués par un système à griffes comportant des griffes formant pince.

3. Dispositif suivant la revendication 1, caractérisé en ce que le transporteur d'amenée est constitué par une bande transporteuse (29; 38) plongeant sous un angle aigu dans le bain de mercure (22), et le transporteur d'évacuation est constitué par une bande transporteuse (33; 40) émergeant sous un angle aigu du bain de mercure (22).

4. Dispositif suivant la revendication 3, caractérisé en ce que la bande transporteuse (29; 38) du transporteur d'amenée et la bande transporteuse (33; 40) du transporteur d'évacuation forment un angle de 20 à 50 degrés, et de préférence un angle de 30 à 45 degrés avec la surface du bain de mercure (22).

5. Dispositif suivant la revendication 3 ou 4, caractérisé en ce qu'à la bande transporteuse (29; 38) du transporteur d'amenée et à la bande transporteuse (33; 40) du transporteur d'évacuation est associée chaque fois, au-dessus de ces dernières, une bande transporteuse supplémentaire (30, 32; 40), étant entendu que les bandes transporteuses associées l'une à l'autre se déplacent de manière synchrone et enserrent les éprouvettes entre elles.

6. Dispositif suivant l'une quelconque des revendications 3 à 5, caractérisé en ce qu'au niveau du transducteur de force (24) est disposée une bande transporteuse horizontale (26) qui reçoit les éprouvettes (5) du transporteur d'amenée (28) et les délivre, après l'opération de mesure, au transporteur d'évacuation (32).

7. Dispositif suivant la revendication 6, caractérisé en ce que la bande transporteuse (26) du transducteur de force (24) ainsi que les paliers et le mécanisme d'entraînement sont tous complètement immergés dans le bain de mercure (22).

8. Dispositif suivant la revendication 7, caractérisé en ce que l'unité immergée dans le bain de mercure (22), comprenant la bande transporteuse (26) ainsi que les paliers et l'entraînement de la bande transporteuse (26) est reliée au dispositif de mesure (23) disposé au-dessus du bain de mercure (22) par l'intermédiaire de tiges (25) relativement minces, qui n'engendrent dès lors, en cas de changement de la profondeur d'immersion, que de très légères variations des forces de flottabilité.

9. Dispositif suivant la revendication 7, caractérisé en ce que l'ensemble du dispositif de mesure et des éléments reliant le transducteur de force au dispositif de mesure est également complètement immergé dans le bain de mercure.

10. Dispositif suivant l'une quelconque des revendications 3 à 5, caractérisé en ce que la bande transporteuse (38) constituant le transporteur d'amenée et la bande transporteuse (40) constituant le transporteur d'évacuation forment un système de transport continu, passant en dessous du transducteur de force, composé de différentes courroies de transport (66), et que le transducteur de force (42) présente, sur sa face inférieure, des évidements (67), dans lesquels les courroies de transport (66) pénètrent après un déplacement vertical relatif entre le transducteur de force (42) et les courroies de transport (66) à la suite du positionnement de l'éprouvette (5) lors de l'opération de mesure, l'éprouvette (5) s'appliquant contre les nervures (68) du transducteur de force (42), qui délimitent les évidements (67).

11. Dispositif suivant l'une ou plusieurs des revendications 1 à 10, caractérisé en ce qu'il est intégré dans une ligne automatique, qui comprend un poste de pesage (17, 18) situé en amont ou en aval pour déterminer le poids de l'éprouvette (5).

12. Dispositif suivant la revendication 11, caractérisé en ce qu'il est intégré dans une ligne automatique, qui comprend, au début de la ligne, un dispositif de coupe automatique (2, 4) destiné à diviser des carreaux de céramique ou des galettes de céramique pressées en éprouvettes individuelles (5).
